(19) Europäisches Patentamt
European Patent Office
Office européen des brevets

(11) Veröffentlichungsnummer: **0 010 294**
**B1**

(12) # EUROPÄISCHE PATENTSCHRIFT

(45) Veröffentlichungstag der Patentschrift: **19.05.82**

(21) Anmeldenummer: **79104000.9**

(22) Anmeldetag: **16.10.79**

(51) Int. Cl.³: **C 07 C 133/00,**
C 07 D 265/30,
C 07 D 295/22
//A61K31/175, A61K31/445,
A61K31/535

(54) **Neue 1-(2-Halogenäthyl)-1-nitrosoharnstoffe und deren Herstellung.**

(30) Priorität: **19.10.78 DE 2845574**

(43) Veröffentlichungstag der Anmeldung:
**30.04.80 Patentblatt 80/9**

(45) Bekanntmachung des Hinweises auf die Patenterteilung:
**19.05.82 Patentblatt 82/20**

(84) Benannte Vertragsstaaten:
**AT BE CH FR GB IT LU NL SE**

(56) Entgegenhaltungen:
**AT - B - 331 808**
**DE - A1 - 2 659 862**
**Chem. Abstracts 82 (1975), 43263y**

(73) Patentinhaber: **Stiftung Deutsches Krebsforschungszentrum**
**Im Neuenheimer Feld 280**
**D-6900 Heidelberg 1 (DE)**

(72) Erfinder: **Eisenbrand, Gerhard, Dr.**
**Banngartenstrasse 19**
**D-6902 Sandhausen (DE)**

(74) Vertreter: **Deufel, Paul, Dr. et al,**
**Patentanwälte Müller-Boré.Deufel Schön.Hertel**
**Siebertstrasse 4**
**D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 294 B1

Courier Press, Leamington Spa, England.

Neue 1-(2-Halogenäthyl)-1-nitroscharnstoffe und deren Herstellung

Die Synthese von unsymmetrisch 1,3-disubstituierten Nitrosoharnstoffen durch Umsetzung von N-Nitroso-N-alkylcarbamoylazid mit einem Diamin, Aminoalkohol oder Aminosäurederivat ist aus der DE—PS 26 23 420 bekannt.

In 3-Stellung substituierte 1-(2-Chloräthyl)-3-nitrosoharnstoffe mit tumorhemmender Wirkung sind bekannt, z.B. aus Chemical Abstracts 82, (1975), 43263y, wo über Methylengruppen an die Nitrosoharnstoffkomponente gebundene Ringsysteme, z.B. Pyridin und Pyrimidin, und deren Wirkung gegen Leukämie L1210 beschrieben werden. Im Test am schwierig zu beeinflussenden Yoshida Sarkom, welches in der Colonwand von Ratten implantiert wurde und ein menschlichen Colontumoren vergleichbares Tumormodell darstellt, erwiesen sich diese Verbindungen jedoch als sehr wenig wirksam. Dabei ist zu berücksichtigen, daß die Mäuseleukämie L1210 vergleichweise leicht zu beeinflussen ist, da meist schon am ersten oder zweiten Tag nach intraperitonealer Applikation der Leukämiezellen die Behandlungen mit den zu testenden Verbindungen erfolgen. Ein wesentlich härteres Tumormodell stellt demgegenüber z.B. auch der Rattenleukämietest L5222 dar, wo meist sehr spät, nämlich am 8. Tage, nach der Inokulation des Tumors, die zu testende Substanz appliziert wird, also zu einem Zeitpunkt, wo davon auszugehen ist, daß schon das Gehirn bzw. die Meningen von Leukämiezellen besiedelt sind.

Es hat sich nun gezeigt, daß durch heterocyclische Ringe oder Alkylreste substituierte Analoga des klinisch etablierten Chemotherapeutikums CCNU (1-(2-Chloräthyl)-1-nitroso-3-(cyclohexyl)-harnstoff) erhalten werden können, die ebenfalls tumorhemmende Wirkung besitzen. Man kann also ausgehend von N-(2-Halogenäthyl)-N-nitrosocarbamoylazid, das ebenfalls in der DE—PS 26 23 420 gezeigt ist, zu einer weiteren Gruppe interessanter Chemotherapeutika gelangen. Bezüglich der Herstellung des Halogenäthyl-N-nitrosocarbamoylazids sei auf die genannte DE—PS verwiesen.

Die Herstellung der hier gezeigten heterocyclischen ringsubstituierten Verbindungen erfolgt mit cyclischen N-Aminoverbindungen (Hydrazinen), wie N-Aminopiperidin, N-Aminomorpholin, N-Amino-2,6-dimethylmorpholin.

Die neuen Verbindungen entsprechen der allgemeinen Formel:

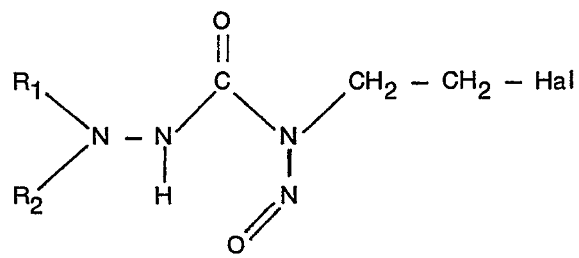

worin $R_1$ und $R_2$ Bestandteil eines gesättigten oder ungesättigten Ringsystems, insbesondere eines 6-gliedrigen Ringsystems sein können, das außerdem weitere Heteroatome, insbesondere Sauerstoff oder Stickstoff, enthalten kann.

Der Ring kann zusätzlich mit kurzkettigen Alkylgruppen oder bis zu 3 Gruppen OH, COOH, COOR, wobei R eine Alkylgruppe mit 1 bis 6 Kohlenstoffatomen bedeutet, mit $CONH_2$ sowie der Chloräthylnitrosoureidogruppe substituiert sein. Der Ausdruck Hal bedeutet Chlor oder Fluor.

$R_1$ und $R_2$ können außerdem H, sowie gerad- oder verzweigtkettiges Alkyl (bis $C_6$) bedeuten, das außerdem mit bis zu 3 OH-Gruppen einer oder mehreren (bis zu 3) $—OSO_2CH_3$, COOH, COOR-Gruppen, wobei R die oben angegebene Bedeutung besitzt oder $CONH_2$ oder Halogen substituiert sein kann.

In diesem Fall geht man von Hydrazinderivaten aus, also acyclischen N-Aminoverbindungen und erhält nicht heterocyclisch ringsubstituierte Verbindungen sondern Hydrazinderivate, nämlich 1-Dialkyl-4-nitroso-4-(2-chloräthyl)-semicarbazide, die ebenfalls eine interessante Gruppe von Chemotherapeutika mit tumorhemmender Wirkung darstellen.

Die folgenden Beispiele erläutern die Erfindung.

Beispiel 1

1-(2-Chloräthyl)-1-nitrosoo-3-(1-piperidino)harnstoff Zu einer Lösung von 0,1 Mol N-(2-Chloräthyl)-N-nitrosocarbamoylazid in 75 ml Dichlormethan wird bei −5°C unter Rühren innerhalb 2 h eine Lösung von 0,2 Mol N-Aminopiperidin in 20 ml eines Gemisches von 4 Volumteilen Dichlormethan und 1 Volumteil n-Pentan hinzugetropft. Nach der Zugabe wird eine Stunde nachgerührt; es fällt ein weißlich-gelber Niederschlag aus. Anschließend wird mit gleichem Volumen 0,1 n $H_2SO_4$ ausgeschüttelt. Die organische Phase wird über $Na_2SO_4$ getrocknet und am Rotationsverdampfer eingeengt. Aus Äthylformiat/n-Pentan werden beim Stehen im Tiefkühlfach gelbe Kristalle erhalten, die nach Umkristallisieren aus dem gleichem Lösungsmittelgemisch dünnschichtchromatographisch einheitlich sind. (Kieselgelfolien, Laufmittel n-Pentan/Diäthyläther/Dichlormethan 5:2:2.)

Ausbeute: 21%; Fp.: 88—89°C;

| Elementaranalyse: | | Ber. (%); | Gef. (%); |
|---|---|---|---|
| | C: | 40,94; | 41,28; |
| | H: | 6,44; | 6,64; |
| | N: | 23,87; | 23,91; |
| | Cl: | 15,10; | 15,17; |

Die spektroskopischen Daten betätigen Einheitlichkeit und Struktur der Verbindung. So zeigt das IR-Spektrum (KBr-Preßling) die typischen Banden bei 3240 $cm^{-1}$ (NH), 1710 $cm^{-1}$ (CO), 1525 $cm^{-1}$ (CNH) und 1480 $cm^{-1}$ (NO). Im NMR-Spektrum ($D_6$-Aceton) erscheint das NH-Signal bei $\delta = 8{,}9$ ppm (breit, $D_2O$-austauschbar), das typische downfield-Triplett des nitrosierten Chloräthylureidostrukturelementes liegt bei $\delta = 4{,}17$ ppm ($CH_2$—NNO) da upfield-Triplett bei $\delta = 3{,}60$ ppm (Cl—$CH_2$). Charakteristische Fragmente im Massenspektrum (70 ev) liegen im oberen Massenbereich bei m/e 204 (M—NO)$^+$ mit dem für Monochlorverbindungen typischen Peakverhältnis von 3:1 zum Isotopenpeak bei m/e 206, bei m/e 126 (M—Cl($CH_2$)$_2$$N_2$OH* und bei m/e 99 (M—$CON_2O(CH_2)_2Cl$). Die Substanz gibt die typische Farbreaktion auf N-Nitrosoverbindungen (G. Eisenbrand, R. Preußmann, Arzneimittelforsch. *20*, 1513 (1970)).

Tierversuchsergebnisse zeigen eine sehr gute Wirksamkeit bei der Rattenleukämie L 5222.

Beispiel 2

1-(2-Chloräthyl)-1-nitroso-3-(4-morpholino)-harnstoff

Zu einer Lösung von 0,1 Mol N-(2-Chloräthyl)-N-nitrosocarbamoylazid in 75 ml Dichlormethan wird bei —5°C unter Rühren innerhalb 2 h eine Lösung von 0,2 Mol N-Aminomorpholin in 20 ml eines Gemisches von 4 Volumteilen Dichlormethan und 1 Volumanteil n-Pentan hinzugetropft. Die weitere Aufarbeitung erfolgt analog Beispiel 1. Aus n-Pentan/Dichlormethan werden schwachgelbe Kristalle erhalten, die nach Umkristallisieren aus Äthylformiat/n-Pentan dünnschichtchromatographisch einheitlich sind (Kieselgel-Folien, Laufmittel analog Beispiel 1).

Ausbeute: 40%; Fp.: 77—78°C;

| Elementaranalyse: | | Ber. (%); | Gef. (%); |
|---|---|---|---|
| | C: | 35,53; | 35,59; |
| | H: | 5,54; | 5,79; |
| | N: | 23,67; | 23,38; |
| | Cl: | 14,98; | 14,75; |

Spektroskopische Daten bestätigen Einheitlichkeit und Struktur der Verbindung. Das IR-Spektrum zeigt für die Nitrosoharnstoffe typische Banden bei 3205 $cm^{-1}$ (NH), 1710 $cm^{-1}$ (CO), 1525 $cm^{-1}$ (CNH) und 1480 $cm^{-1}$ (NO). Im NMR-Spektrum ($D_6$-Aceton) liegt das NH-Signal bei $\delta = 9{,}1$ ppm (breit, $D_2O$-austauschbar), das downfield-Triplett des nitrosierten Chloräthylstrukturelements liegt bei $\delta = 4{,}17$ ppm ($CH_2$—NNO), das upfield-Triplett, teilweise überlappend mit den Signalen der $CH_2$-Gruppen in Stellung 2 und 4 des Morpholinringes, liegt bei $\delta = 3{,}60$ ppm (Cl—$CH_2$). Charakteristische Fragmente im Massenspektrum (70 ev) liegen bei m/e 206 (M—NO)$^+$, mit dem für Monochlor-Verbindungen typischen Peakverhältnis von 3:1 zum Isotopenpeak bei m/e 208, bei m/e 128 (M—Cl—($CH_2$)$_2$$N_2$OH)$^+$ und bei m/e 101 (M—$CON_2O(CH_2)_2Cl$)$^+$. Die Substanz gibt die typische Farbreaktion auf N-Nitrosoverbindungen.

Tierversuchsergebnisse zeigen sehr gute Wirksamkeit bei der Pattenleukämie L 5222. Die Verbindung ist im Gegensatz zur klinisch etablierten Substanz CCNO sehr gut wasserlöslich. Sie ist zur Zeit in Erprobung am in das Colon implantierten Yoshida Sarkom der Ratte und zeigt dort bessere therapeutische Wirksamkeit als die klinisch bei Colontumoren eingesetzte Verbindung Methyl-CCNU.

Beispiel 3

1-(2-Chloräthyl)-1-nitroso-3-(4-(2,6-dimethyl-morpholino))-harnstoff

Zu einer Lösung von 0,02 Mol N-(2-Chloräthyl)-N-nitrosocarbamoylazid in 15 ml Dichlormethan wird bei —5°C unter Rühren innerhalb 2 h 0,04 Mol 4-Amino-2,6-dimethylmorpholin in 20 ml Dichlormethan hinzugetropft. Nach weiterem einstündigem Rühren wird weiteraufgearbeitet wie in Beispiel 1.

Aus Dichlormethan/n-Pentan werden schwach-gelbe Kristalle erhalten, die nach Umkristallisation dünnschichtchromatographisch einheitlich sind.

Ausbeute: 45% d.M.; Fp: 102—104°C

Spektroskopische Daten bestätigen Einheitlichkeit und Struktur der Verbindung.

Das IR-Spektrum (KBr-Preßling) zeigt die typischen Nitrosoharnstoffbanden (NH, CO, CNH, NO). Im NMR-Spektrum ($D_6$-Aceton) erscheint das NH-Signal bei $\delta = 9$ ppm, das typische downfield Triplett des nitrosierten Chloräthylnitrosoureidostrukturelements liegt bei $\delta = 4{,}17$ ($CH_2$—NNO), das upfield Triplett, teilweise überlagert von den Ring-CH-Signalen in Stellung 2 und 6, liegt bei $\delta = 3{,}60$ ppm. Das Signal der Methylgruppen in Stellung 2 und 6 des Morpholinringes erscheint bei $\delta = 1{,}15$ ppm. Die Substanz gibt die typische Farbreaktion auf N-Nitrosoverbindungen.

Beispiel 4

1-(2-Chloräthyl)-1-nitroso-3-dimethylaminoharnstoff

In einer Lösung von 0,05 Mol N - (2 - Chloräthyl) - N - nitrosocarbamoylazid in 15 ml Isopropanol/Tetrachlorkohlenstoff wird bei —10°C unter Rühren 0,1 Mol Dimethylhydrazin in 10 ml Isopropanol so langsam zugetropft, daß der pH-Wert nicht über 7,5 ansteigt (3 h Reaktionszeit). Nach weiterem einstündigem Rühren wird mit verdünnter Schwefelsäure angesäuert und mit Äthylformiat ausgeschüttelt. Die organische Phase wird über $Na_2SO_4$ getrocknet und eingeengt. Das Rohprodukt wurde durch Säulenchromatographie an einer Silicagel-Fertigsäule (Laufmittel Äther(Äthanol 98:2) gereinigt. Nach Abziehen des Lösungsmittels blieb ein öliger Rückstand der dünnschichtchromatographisch einheitlich war.

Ausbeute: 20%

Das Kernresonanzspektrum ($D_6$-Aceton) bestätigt Einheitlichkeit und Struktur der Verbindung. Das NH-Signal erscheint bei 8,9 ppm, das downfield Triplett des nitrosierten Chloräthylnitrosoureidostrukturelementes liegt bei $\delta = 4{,}17$ ($CH_2NNO$), das upfield Triplett bei $\delta = 3{,}60$ ppm. Das Signal der Methylgruppen erscheint bei $\delta = 2{,}75$ ppm. Die Substanz gibt die typische Farbreaktion auf N-Nitrosoverbindungen.

**Patentansprüche**

1. 1,3-disubstituierte Nitrosoharnstoffe der allgemeinen Formel

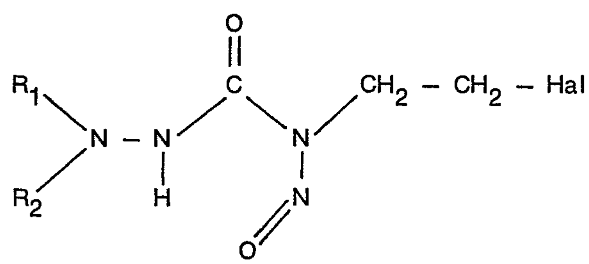

worin $R_1$ und $R_2$ H oder gerad- oder verzweigkettiges Alkyl bedeuten, das außerdem mit bis zu 3 Resten OH, COOH, COOR oder $CONH_2$ substituiert sein kann, wobei R eine Alkylgruppe mit 1 bis 6 Kohlenstoffatomen bedeutet, oder worin $R_1$ und $R_2$ Bestandteile eines gesättigten oder ungesättigten Ringsystems, insbesondere eines 6-gliedrigen Ringsystems sein können, das außerdem weitere Heteroatome, insbesondere Sauerstoff oder Stickstoff, enthalten kann und wobei der Ring zusätzlich mit kurzkettigen Alkylgruppen oder bis zu 3 Gruppen OH, COOH, COOR, wobei R die oben angegebene Bedeutung besitzt, $CONH_2$, Hal oder mit der Chloräthylnitrosoureidogruppe substituiert sein kann und Hal Chlor oder Fluor darstellt.

2. Verfahren zur Herstellung der Verbindung nach Anspruch 1, dadurch gekennzeichnet, daß man N-(2-Halogenäthyl)-N-nitroso-carbamoylazid mit einem Hydrazinderivat oder einer cyclischen N-Aminoverbindung in einem gegen die Reaktionsteilnehmer inerten Lösungsmittel in an sich bekannter Weise reagieren läßt.

3. Verfahren nach Anspruch 2, dadurch gekennzeichnet, daß man als N-2-Halogenäthylverbindung die Chloräthyl- oder Fluoräthylverbindung einsetzt.

4. Verfahren nach Anspruch 2 oder 3, dadurch gekennzeichnet, daß man als cyclische N-Aminoverbindung N-Aminopiperidin oder N-Aminomorpholin, N-Amino-2,6-dimethylmorpholin und deren Derivate einsetzt.

**Revendications**

1. Nitroso urées 1, 3-disubstituées de la formule générale

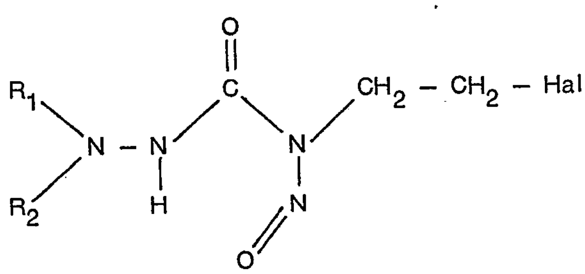

dans laquelle $R_1$ et $R_2$ signifient H ou de l'alcoyle à chaîne droite ou ramifiée pouvant être, en outre, substitué avec jusqu'à trois restes OH, COOH, COOR ou $CONH_2$, R signifiant un group d'alcoyle comportant 1 à 6 atomes de carbone, ou dans laquelle $R_1$ et $R_2$ peuvent être des constituants d'un système cyclique saturé ou non saturé, en particulier d'un système cyclique à 6 membres, pouvant en outre comporter d'autres hétéroatomes, en particulier de l'oxygène et de l'azote, et le cycle pouvant additionnellement être substitué par des groupes d'alcoyle à chaîne courte ou jusqu'à 3 groupes OH, COOH, COOR, où R a la signification susdite, par $CONH_2$, Hal ou par le groupe chloréthylenitrosouréides, et Hal représentant du chlore ou du fluor.

2. Procédé de préparation du composé suivant la revendication 1, caractérisé en ce que l'on fait réagir du N-(2-halogénure d'éthyle)-N-nitroso-carbamoylacide avec un dérivé d'hydrazine ou un composé N-amino cyclique, dans un solvant inerte pour les éléments participant à la réaction, de façon connue en soi.

3. Procédé suivant la revendication 2, caractérisé en ce que l'on utilise, en tant que composé N-2-halogénure d'éthyle, le composé chlorure d'éthyle ou fluoréthyle.

4. Procédé suivant la revendication 2 ou 3, caractérisé en ce que l'on utilise, en tant que composé cyclique N-amino, de 1'N-aminopipéridine ou N-aminomorpholine, N-amino-2,6-diméthylemorpholine et leurs dérivés.

**Claims**

1. 1,3-disubstituted nitrosoureas of the general formula:

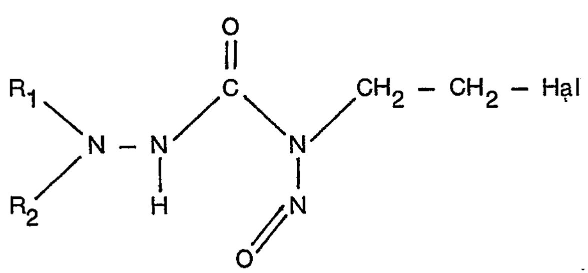

wherein $R_1$ and $R_2$ are H or straight chained or branched alkyl, which can be substituted in addition with up to 3 radicals OH, COOH, COOR or $CONH_2$ where R denotes an alkyl group with 1 to 6 carbon atoms or wherein $R_1$ and $R_2$ can be components of a saturated or unsaturated ring system, particularly of a 6-member ring system, which can also contain other heteroatoms, particularly oxygen or nitrogen, and where the ring can be substituted additionally with up to 3 groups OH, COOH, COOR, where R has the above indicated meaning, $CONH_2$, Hal, or with the chloroethyl nitrosoureido-group, and Hal denotes chlorine or fluorine.

2. Method for the preparation of the compound according to claim 1, characterized in that N-(2-halogenethyl)-N-nitrosocarbamoylazide is reacted in a known per se manner with a hydrazine derivative or a cyclic N-amino-compound in a solvent that is inert to the reaction partners.

3. Method according to claim 2, characterized in that the chloroethyl- or fluoroethyl compound is used as a N-2-halogenethyl compound.

4. Method according to claim 2 or 3, characterized in that N-aminopiperidine or N-aminomorpholine, N-amino-2,6 dimethylmorpholine and their derivatives are used as a cyclic N-amino-compound.